Europäisches Patentamt

European Patent Office

Office européen des brevets

(19)

(11) Veröffentlichungsnummer: **0 303 557**
**A2**

(12) **EUROPÄISCHE PATENTANMELDUNG**

(21) Anmeldenummer: **88730165.3**

(22) Anmeldetag: **21.07.88**

(51) Int. Cl.⁴: **A 61 M 1/00**

(30) Priorität: **21.07.87 DE 8710130**

(43) Veröffentlichungstag der Anmeldung:
**15.02.89 Patentblatt 89/07**

(84) Benannte Vertragsstaaten: **DE ES FR GB**

(71) Anmelder: **Effner GmbH**
**Alt-Lankwitz 102 .**
**D-1000 Berlin 46 (DE)**

(72) Erfinder: **Vaubel, Wolf-Ekkehard, Prof. Dr. med.**
**Steinäckerstrasse 15**
**D-1000 Berlin 45 (DE)**

**Anapliotis, Emmanuel**
**Kiebitzweg 5**
**D-1000 Berlin 33 (DE)**

(74) Vertreter: **Christiansen, Henning, Dipl.-Ing.**
**Patentanwalt CHRISTIANSEN Pacelliallee 43/45**
**D-1000 Berlin 33 (DE)**

(54) **Sauger.**

(57) Operationssauger zum Absaugen von Flüssigkeiten bei chirurgischen Eingriffen mit einem von Hand in das Operationsfeld einzuführenden Saugrohr, mit einem Griffteil das aufweist: einen inneren durchgehenden Kanal, welcher mit einem absperrbaren flexiblen Schlauchstück versehen ist, ein Saugrohr und ein Unterdruckanschluß, ein Betätigungselement zum Absperren des flexiblen Schlauchstücks, welches am saugrohrseitigen Ende des des Griffteils gelagert ist, mit einem Federelement, welches sich an einem Widerlager innerhalb des Griffteils abstützt und in der entspannten Stellung der Feder den Schlauchquerschnitt unter Einschluß eines Klemmelements nach Art einer Schlauchklemme absperrt, einem Verbindungselement zum Zusammenpressen des Federelements, das mit dem Betätigungselement in mechanischer Verbindung steht.

· Fig. 1

EP 0 303 557 A2

**Beschreibung**

## Sauger

Die Erfindung betrifft einen Sauger, insbesondere zur chirurgischen Anwendung, der im Oberbegriff des Anspruchs 1 angegebenen Art.

Die bekannten Sauger sind konstruktiv aufwendig und erfordern insbesondere im Absperrbereich spezielle Dichtungsteile, welche nur kompliziert herstellbar sind und im Falle ihres Verschleißes besondere Ersatzteile erforderlich machen.

Ein derartiger Sauger ist aus der DE-PS 33 07 517 bekannt. Nachteilig dabei ist ferner, daß die Betätigungskräfte vom Unterdruck und der Öffnungsstellung der Absperrung abhängig sind. Außerdem liegt der Sauger schlecht in der Hand.

Der im Anspruch 1 angegebenen Erfindung liegt die Aufgabe zugrunde, einen gattungsgemäßen Sauger zu schaffen, welcher in der Herstellung einfach ist, mit konstruktiv einfachen Absperrelementen arbeitet und gut handhabbar ist.

Der Sauger weist ein elastisches Schlauchstück auf, das innerhalb eines vorzugsweise zylindrischen Griffteils gehalten ist, wobei ein federbelastetes Betätigungselement in seinem entspannten Zustand mit einem Druckstück auf den flexiblen Schlauch einwirkt und den Schlauchquerschnitt absperrt, wobei das Betätigungselement eine Drucktaste aufweist.

Besonders vorteilhaft bei der erfindungsgemäßen Lösung ist, daß sich bei konsequenter Anwendung der auch den Weiterbildungen zugrundeliegenden Grundsätze eine Anordnung ergibt, welche eine schlanke Konstruktion ermöglicht, die im wesentlichen keine vorspringenden Teile aufweist und einfach herzustellen ist, so daß der Sauger insbesondere als Einwegartikel geeignet ist. Er ist auch vorteilhaft in Verbindung mit evakuierten oder evakuierbaren Behältnissen verwendbar, die jeweils ein vorgegebenes Saugvolumen bereithalten, so daß für bestimmte Eingriffe oder in Notfallsituationen nicht eine vollständige Unterdruckversorgung vorhanden sein muß.

Die Anwendung ist in all denjenigen Fällen vorteilhaft, bei denen in möglichst einfacher Weise ein Flüssigkeitsvolumen abgesaugt werden soll.

Wenn gemäß bevorzugter Weiterbildung der Erfindung das Verbindungselement eine Überbrückung des Schlauches bildet und das Betätigungselement als Drucktaste mit Betätigungsrichtung senkrecht zum Griffteil ausgebildet ist, so ergibt sich eine einfache und ergonomische Handhabung.

Indem der flexible Schlauch mit dem Saugrohr und/oder dem Unterdruckanschluß mittels koaxialer Steckverbindung über jeweils ein Zwischenstück verbunden ist, ist - bei geeignetem Querschnitt der Verbindung - das Durchgangsvolumen innerhalb des Operationssaugers sehr klein, so daß auch ein nur begrenztes Saugvolumen auf der Unterdruckseite nahezu vollständig ausgenutzt werden kann.

Wenn der Saugrohr- bzw. Unterdruckanschluß durch ein Zwichenstück aus bevorzugt flexiblem Material gebildet wird und einen, gegebenenfalls gestuften, zylindrischen Anschlußstutzen aufweist, ist das Gerät in vielfacher Weise mit geeigneten Anschlußstücken kombinierbar. Für eine einfache Montage ist es günstig, wenn in Durchlaßrichtung aufeinanderfolgende Teile mit koaxialen Steckverbindungen versehen sind, welche einen inneren Durchlaß aufweisen. Die Zwischenstücke als Verbinder zwischen Schlauchstück und den externen Teilen können entweder an das Griffteil angeschlossen werden und sind dann bevorzugt auf dessen Endteile aufgesteckt oder werden in Halbschalen des Griffteils vor deren Zusammenfügen eingelegt. Die Zwischenstücke enthalten also zum Inneren des Griffes hin die Anschlüsse für das Schlauchstück, wobei dieses auf entsprechende Anschlußstutzen auf- oder in geeignet vorgesehene Ausnehmungen eingeschoben werden kann. Die nach außen gerichteten Enden der Zwischenstücke dienen als externe Anschlüsse und können entsprechend als Schlauchanschlüsse oder zum Einsetzen von Kanülen etc. ausgebildet sein. Eine bevorzugte Ausführung weist einen sogenannten Lue-Lock-Anschluß auf der Seite zum Anschluß des Saugrohrs, also der Arbeitsseite des Griffteils, auf. Wenn die Zwischenstücke aus einem flexiblen Material bestehen übernehmen sie gleichzeitig die Abdichtung zu den extern angeschlossenen Verbindungselementen.

Günstig für die Montierbarkeit ist ferner, wenn das Schlauchstück in einem Bereich angeordnet ist, welcher eine das Griffstück durchquerende Kammer bildet, wobei in die Kammer das Schlauchstück mitsamt vormontiertem Anschlußstutzen oder Zwichenstücken einfügbar ist. Eine derartige "Kammer" wird beispielsweise durch zwei Gehäusehalbschalen gebildet.

Bei einer anderen bvorzugten Ausführung ist das Schlauchstück durch das gesamte Griffteil hindurchgeführt und an den Enden nach außen "umgestülpt", so daß der umgestülpte Bereich des Schlauches gleichzeitig eine Dichtung für ein auf das Griffstück aufzusteckendes externes Element bildet.

Für eine kostengünstige Herstellung als Einwegteil ist es besonders vorteilhaft, wenn die Kammer eine erste Gehäusehalbschale bildet und das Abdeckteil als zweite Gehäusehalbschale ausgebildet ist, wobei wegen der Formkosten die beiden Gehäusehalbschalen im wesentlichen übereinstimmend ausgebildet sind. Die Kammer und das Abdeckteil bzw. die Gehäusehalbschalen werden mittels Klemmstiften und an diese angepaßte Ausnehmungen (bzw. Krallen und an diese angepaßte Hinterschneidungen) einfach ineinandergerastet, nachdem die innere Verbindungsleitung mit dem absperrbaren Schlauchstück einschließlich zugehöriger Betätigungs- und Verbindungselemente eingelegt wurde. Dazu sind bevorzugt innerhalb der Gehäusehalbschalen Stege als Lagerung vorgesehen.

Um ein mögliches Verkleben der einander gegenüberliegenden Innenflächen des Schlauchstücks bei entspanntem Zustand des Federelements zu verhindern, wird es bei einer bevorzugten Ausführung einer Verpackung durch deren Bemessung unter Vorspannung gehalten, so daß das Betätigungselement bei in der Verpackung befindlichem Operationssauger niedergedrückt ist und somit das Schlauchstück mindestens teilweise geöffnet ist.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 ein erstes Ausführungsbeispiel im Längsschnitt,

Figur 2 das Ausführungsbeispiel in verpacktem Zustand im Querschnitt,

Figur 3 eine Variante des Ausführungsbeispiels gemäß Figur 1 in einer Verpackung im Längsschnitt,

Figur 4 die Variante gemäß Figur 2 ohne Verpackung sowie

Figur 5 eine weitere Variante des Ausführungsbeispiels gemäß Figur 1 im Querschnitt.

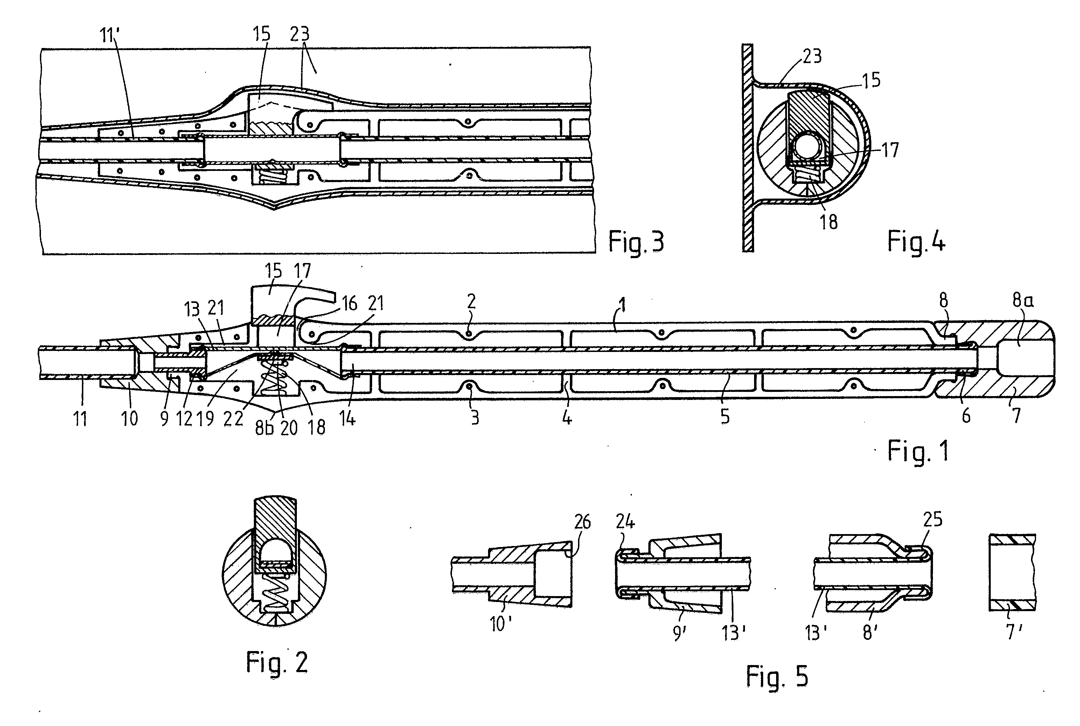

Bei dem in Figur 1 dargestellten Ausführungsbeispiel besteht der wesentliche Teil des Instruments aus einem hohlen Griffteil 1, der in zwei Hälften als Kunststoffspritzteil ausgebildet ist. In der Draufsicht ist das Gehäuse geöffnet wiedergegeben, wobei die dem Betrachter zugewandte Gehäusehälfte fortgelassen ist, um den Einblick in das Innere freizugeben.

Beide Hälften werden an der Trennstelle mit Krallen oder Stiften verbunden, die in entsprechende Aussparungen der Gegenseite eingreifen. Klemmstiften und entsprechenden Ausnehmungen zu deren Aufnahme 2 bzw. 3) - bzw. Krallen und entsprechende Hinterschneidungen -, sind jeweils wechselweise angebracht. Auf diese Weise sind die beiden Hälften des Gehäuses nahezu identisch ausgebildet und können mit derselben (beispielsweise zur Erzeugung eines Durchbruchs für das Betätigungselement) geringfügig veränderbaren Form hergestellt werden.

Rippen 4 halten ein inneres Rohr 5 räumlich fixiert, welches als Unterdruckleitung dient. Das hintere vorstehende Ende des Rohrs bildet einen Stutzen 6, auf den ein Zwischenstück darstellender Auslaßverbinder 7 aufgesteckt ist. Der Auslaßverbinder ist mit seiner Durchlaßöffnung sowohl über den vorstehenden Stutzen 6 als auch über eine stutzenförmige Anformung 8 des Griffteils 1 gestülpt. Durch den Auslaßverbinder werden die beiden Hälften des Griffteils zusätzlich zusammengehalten.

Das Auslaßende weist bei insgesamt im wesentlichen hohlzylindrischer Formgebung eine Auslaßöffnung 8a mit zylindermantelförmiger Wandung auf, in die ein Schlauchende eingeführt und befestigt werden kann. Zur Erhöhung der Sicherheit der Anschlußverbindung können noch ringförmige Profilierungen oder dergleichen (in der Zeichnung nicht dargestellt) an der Innenseite der hohlzylindrischen Wandung vorgesehen sein.

Die Außenform des Griffteils erweitert sich zunächst nach Art eines Kegelstumpfes mit im Querschnitt konkav geformter Mantellinie zu einem Bereich 8b maximalen Querschnitts, um sich anschließend wieder entsprechend zu verjüngen. Dann erfolgt eine Stufung zu einem Stutzen 9 mit zylindrischer Außenwandung, auf den ein Verbindungsstück 10 - ebenfalls als Zwischenstück - aufgeschoben ist, welches ein Saugrohr 11 trägt.

Der Stutzen 9 bildet dabei die Anschlußstelle für ein Verbindungssystem zum Ansetzen unterschiedlicher Saugelemente. In den zylindrischen Innenquerschnitt des Stutzens 9 ist ein weiterer zylindrischer Stutzen 12 eingefügt, der bevorzugt aus Glas oder Kunststoff gefertigt ist, während das Gehäuse 1 aus Kunststoff besteht. Der Stutzen 12 weist nach innen - in eine Ausnehmung 19 des Gehäuses 1 - gerichtet einen Schlauchanschlußstutzen mit einer Erweiterung des Endquerschnitts zur Aufnahme eines Silikonschlauchs auf.

Bei - in der Zeichnung nicht dargestellten - anderen bevorzugten Ausführungen der Erfindung ist das Schlauchstück mit dem jeweils ein Zwischenstück bildenden Auslaßverbinder 7 bzw. dem Verbindungsstück 10 direkt verbunden, welche dazu entsprechende Anschlußstutzen oder Ausnehmungen unmittelbar angeformt enthalten. Diese Zwischenstücke können alternativ auch in die Endbereiche der Griffteile eingelegt sein, deren Halbschalen sie bei der Montage beidseits umgreifen und im zusammengesetzten Zustand des Griffs arretieren. Das Verbindungsstück 10 weist bevorzugt an der Außenseite einen sogenannten Lue-Lock-Anschluß auf.

Auf den Stutzen 12 ist ein Silikonschlauch 13 aufgeschoben, dessen anderes Ende auf einem Stutzen 14 befestigt ist, welcher an das Rohr 5 anschließt. Die Unterdruckverbindung durchquert damit den Innenraum des Gehäuses 1 in Längsrichtung vollständig.

Die Anordnung bildet damit einen Operationssauger, der an einem Griffteil 1 gehalten und sich insbesondere mittels der sich durch die konkav-kegelstumpfförmige Formgebung mit maximalem Querschnitt (Kante 8b) auch nach Art eines Schreibgerätes führen läßt. Der Daumen oder Zeigefinger kommt dabei im Bereich der Kante 8b zu liegen. Hier bildet eine Taste 15 ein Element, welches durch Druck betätigbar ist und bei einer derartigen Druckbetätigung den Schlauch 13 öffnet. Dazu wird die Taste in einer ihrem Außenquerschnitt angepaßten Aussparung 16 des Gehäuses 1 geführt, in das der bei fehlendem äußeren Druck herausstehende Bereich der Taste versenkbar ist. In den Querschnittszeichnungen ist diese Position in Figur 2 bzw. 3 dargestellt.

Die Taste 15 weist eine in Längsrichtung durchgehende Ausnehmung 17 zur Aufnahme des Schlauchs 13 auf, welche an der unteren, einer Schraubenfeder 18 zugewandten Seite abgeflacht ist. Die Schraubenfeder 18 stützt sich einerseits in einer unteren Ausnehmung des Gehäuses 1 ab, dessen Innenseite ein Widerlager für die Feder 18 bildet, und wird an ihrem anderen Ende an einem Nippel 20 der Drucktaste 15 zentriert.

Der der Ausnehmung 17 benachbarte Teil der Taste 15 überbückt das Schlauchstück 13 und bildet damit ein "Verbindungselement". Im entspannten Zustand der Feder wird die Taste 15 angehoben und ragt aus dem Gehäuse 1 heraus. Der Schlauch wird durch Quetschung der unteren Abflachung geschlossen. Außerhalb des Bereichs, in dem der die Ausnehmung 17 aufweisende Teil der Taste 15 geführt ist, bilden entsprechend geformte Gegenkanten 21 ein Widerlager für den Schlauch, so daß dieser sicher abgesperrt wird. Eine quer zur Längsrichtung des Schlauchs 13 verlaufende wall artige oder prismatische Erhebung 22 auf dem der Drucktaste gegenüberliegenden abgeflachten Bereich der durchgehenden Ausnehmung erhöht den lokalen Anpreßdruck und damit den Quetschdruck auf den Schlauch. Der die Erhebung 22 aufweisende Teil der Taste 15 bildet ein "Klemmelement". Bei einer anderen nicht dargestellten Ausführung ist in günstiger Weise die Gehäuseinnenwandung so ausgebildet, daß sich bei losgelassener Taste 15 die Erhebung an einen entsprechenden gegenüberliegenden Bereich der Wandung anlegt und hier das Schlauchstück direkt einquetscht.

Die Schraubenfeder 18 besteht insbesondere aus Federstahl oder aus Kunststoff. Die letztgenannte Ausführung ist bevorzugt einstückig mit der Taste bzw. einem Verbindungselement ausgebildet. Als Federelement können dabei auch andere Formteile dienen, die mäanderähnlich oder S-förmig (vorzugsweise symmetrisch) an der Innenseite des Gehäuses gleitend oder abrollend ausgebildet sind. Auch eine blattfederartige Konstruktion aus flexiblem Kunststoff ist günstig, bei der sich die freien Enden an der Gehäuseinnenwand abstützen, während der Mittelbereich einstückig mit der Taste 15 verbunden ist.

Die Montage erfolgt in einfacher Weise durch Einlegen der komplett vormontierten inneren Teile mit allen Elementen der Unterdruckverbindung sowie dem Betätigungselement und anschließendes Zusammendrücken der beiden Gehäusehälften.

Figur 2 zeigt die Anordnung gemäß Figur 1 im Querschnitt.

In Figur 3 ist ein Teilbereich einer Variante dargestellt, die mit einem festen Saugstück 11' versehen ist, der di rekt in den vorderen Bereich des Griffeils 1' eingespannt ist. Um ein Verkleben der einander gegenüberliegenden Flächen des Silikon- oder anderen Kunststoffschlauches zu verhindern, wird die Drucktaste 15 von der Verpackung 23 unter Vorspannung gehalten, so daß die Taste mindestens soweit eingedrückt ist, daß der Schlauchquerschnitt mindestens geringfügig geöffnet ist. Die Verpackung besteht als Formpackung insbesondere aus Styropor, tiefgezogener Kunststoffolie oder ist als Blisterpackung ausgebildet.

In Figur 4 ist die Ausführung gemäß Figur 3 (geöffnete Position) im Querschnitt dargestellt, wobei ersichtlich ist, daß durch Eindrücken der Taste der Querschnitt des Schlauches zwischen abgeflachtem Bereich der Ausnehmung 17 und dem Widerlager 21 sich erweitert und der Schlauch nahezu seinen maximalen Kreisquerschnitt einnimmt. Die Schraubenfeder 18 ist dagegen komprimiert.

Durch Druck auf die Taste 15 läßt sich der Öffnungsquerschnitt des Schlauchs in einem weiten Bereich fein steuern, wobei durch Loslassen der Taste ein sofortiges sicheres Verschließen gewährleistet ist.

In den in Figur 5 dargestellten Detaildarstellungen eines weiteren Ausführungsbeispiels kann die Ausführung der nicht vollständig dargestellten Elemente im wesentlichen denjenigen des zuvor dargestellten Ausführungsbeispiels entsprechen. Wichtig ist hierbei das Hinausführen des inneren Schlauchs bis zu den Enden.

Bei diesem Ausführungsbeispiel ist der flexible Schlauch 13' absperrbar und von einem Ende zum anderen des Hand stücks durchgehend ausgebildet. Auf diese Weise ist es möglich, zusätzliche Übergangs- und Dichtungselemente an den Enden zwecks Abdichtung benachbarter Teile einzusparen. Die Dichtungsmaßnahmen bestehen bei diesem Ausführungsbeispiel in vorteilhafter Weise darin, daß die Enden des Schlauches, welche mit 24 und 25 bezeichnet sind, den Abformungen bzw. Stutzen 8' und 9' übergestülpt sind, so daß sie einen zylindrischen nach außen weisenden Bereich aus elastischem Material im Bereich der Enden aufweisen.

Diese zylindrischen Außenflächen sind Dichtungsanschlüsse für die Anschlußelemente 7' bzw. 10', wobei das Element 7' bevorzugt einen beweglichen Schlauch bildet, welcher mit der Unterdruckeinrichtung verbunden ist. Das Element 10' stellt beispielsweise das Ende eines Saugrohres dar, wobei an der Innenseite an dem dem Griffstück zugewandten Ende eine zylindrische Dichtfläche 26 vorgesehen ist, welche den Außenabmessungen der Umstülpung 24 entspricht. Damit kann das Element 10' ohne weiteres auf die Umstülpung 24 aufgeschoben werden und wird dort aufgrund der Haftreibung sicher festgehalten. Um ein unbeabsichtigtes Lösen der Teile 7' bzw. 10' zu vermeiden, können zusätzlich auf den zylindrischen Außenflächen der Ansätze tangential verlaufende Rillen vorgesehen sein, welche ein Abrutschen der aufgesteckten Elemente bzw. der übergestülpten Schlauchenden verhindern.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

**Patentansprüche**

1. Sauger, insbesondere zum Absaugen von Flüssigkeiten bei chirurgischen Eingriffen, mit einem von Hand in das Operationsfeld einzuführenden Saugrohr, mit einem Griffteil das aufweist:

einen inneren durchgehenden Kanal, welcher mit einem absperrbaren flexiblen Schlauchstück versehen ist,

ein Saugrohr und einen Unterdruckanschluß,

ein Betätigungselement zum Absperren des flexiblen Schlauchstücks, welches am saugrohrseitigen Ende des des Griffteils gelagert ist, **gekennzeichnet durch**

ein Federelement (18), welches sich an einem Widerlager innerhalb des Griffteils (1) abstützt und in der entspannten Stellung der Feder den Schlauchquerschnitt unter Einschluß eines Klemmelements nach Art einer Schlauchklemme absperrt,

ein Verbindungselement, welches mit dem Klemmelement und/oder dem Federelement einerseits für deren Bewegung in eine gespannte Stellung des Federelements mit dem Betätigungselement (15) andererseits in mechanischer Wirkverbindung steht.

2. Sauger nach Anspruch 1, **dadurch gekennzeichnet,** daß das Verbindungselement eine Überbrückung des Schlauchstückes (13) bildet und/oder mindestens zwei der Elemente Betätigungselement, Verbindungselement, Klemmelement und/oder Federelement gemeinsam einstückig ausgebildet sind.

3. Sauger nach einem der vorangehenden Ansprüche, **dadurch gekennzeichnet,** daß das Klemmelement ein dem Schlauchstück (13) zugewandtes dachkantartiges Prisma (22) aufweist, welches quer zu dessen Längsachse gerichtet ist.

4. Sauger nach einem der vorangehenden Ansprüche, **dadurch gekennzeichnet,** daß das Betätigungselement als Drucktaste (15) mit Betätigungsrichtung senkrecht zur Achse des Griffteils (1) ausgebildet ist.

5. Sauger nach einem der vorangehenden Ansprüche, **dadurch gekennzeichnet,** daß das Schlauchstück mit einem Saugrohr (11) und/oder einem Unterdruckanschluß (8a) mittels eines Zwischenstücks in Form eines Stutzens (6, 12) und/oder eines zylindrisch durchbohrtes Verbinderelements (7, 10) aus flexiblem Material verbunden ist, welches einen Teil des Griffteils (1) bildet oder in dieses eingefügt ist.

6. Sauger nach einem der vorangehenden Ansprüche, **dadurch gekennzeichnet,** daß in Saugrichtung aufeinanderfolgende Teile von Saugrohr und Unterdruckanschluß und/oder Zwischenstücke und/oder mindestens ein Ende des Schlauchstücks koaxial aufeinandergesteckt sind.

7. Sauger nach einem der vorangehenden Ansprüche, **dadurch gekennzeichnet,** daß das Schlauchstück (13) im wesentlichen das gesamte Griffteil (1) durchquert und insbesondere über mindestens ein Ende des Griffteils hinausragt und um das hohlzylindrische Ende herumgelegt ist, so daß das betreffende Ende (24, 25) des Schlauchstücks im Endbereich den Außenmantel des Hohlzylinders bildet.

8. Sauger nach einem der vorangehenden Ansprüche, **dadurch gekennzeichnet,** daß zwei das Griffteil (1) bildende Gehäusehalbschalen im wesentlichen übereinstimmend ausgebildet sind.

9. Sauger nach einem der vorangehenden Ansprüche, **dadurch gekennzeichnet,** daß das Schlauchstück (13) in einem Bereich des Griffteils angeordnet ist, welcher eine Kammer bildet, wobei insbesondere das Schlauchstück mitsamt vormontiertem mindestens einem Zwischenstück in die Kammer einfügbar und die Öffnung mit einem Abdeckteil verschließbar ist und/oder die Kammer in nerhalb einer ersten Gehäusehalbschale vorgesehen ist und das Abdeckteil als zweite Gehäusehalbschale ausgebildet ist, welche ebenfalls einen Teil der Kammer umschließt.

10. Sauger nach einem der vorangehenden Ansprüche, **dadurch gekennzeichnet,** daß zwei das Griffteil bildende Gehäusehalbschalen mittels Klemmstiften und an diese angepaßte Ausnehmungen (2 bzw. 3) und/oder Krallen und an diese angepaßte Hinterschneidungen zusammenfügbar sind.

11. Sauger nach einem der vorangehenden Ansprüche, **dadurch gekennzeichnet,** daß das Schlauchstück (13) aus Silikon besteht.

12. Verpackung für einen Sauger nach einem der vorangehenden Ansprüche, **dadurch gekennzeichnet,** daß die Verpackung (23) derart bemessen ist, daß das Betätigungselement bei in der Verpackung befindlichem Sauger niedergedrückt und somit das Schlauchstück mindestens teilweise geöffnet ist.

Fig. 3

Fig. 4

Fig. 1

Fig. 2

Fig. 5

EP 0 303 557 A2